# EUROPEAN PATENT APPLICATION

(11) **EP 4 119 945 A1**
(43) Date of publication of application: **18.01.2023**
(21) Application number: 21198212.9
(22) Date of filing: 22.09.2021
(51) Int. Cl.: G01N 33/543, G01N 33/58

(54) **HIGHLY SENSITIVE IMMUNOCONJUGATE, PREPARING METHOD THEREOF, IN VITRO DIAGNOSTIC REAGENT AND IN VITRO DIAGNOSTIC KIT INCLUDING THE SAME**

(30) Priority: 13.07.2021 KR 20210091864
(71) Applicant: Biometrix Technology Inc., Gangwon-do 24232 (KR)
(72) Inventor: KIM, Taisun, 24375 Gangwon-do (KR); SONG, Keumsoo, 24419 Gangwon-do (KR); KIM, Junghun, 03081 Seoul (KR)
(74) Representative: Witte, Weller & Partner Patentanwälte mbB

(57) **Abstract**

Disclosed are a highly sensitive immunoconjugate, and an in vitro reagent and an in vitro diagnostic kit including the same, in which binding specificity with a target substrate is increased and a detection signal is amplified, thereby improving largely the sensitivity, accuracy and reproducibility of detection.

## Description

### Technical Field

The present disclosure relates to a highly sensitive immunoconjugate, and an in vitro diagnostic reagent and an in vitro diagnostic kit including the same. More particularly, the present disclosure relates to a highly sensitive immunoconjugate, and an in vitro diagnostic reagent and an in vitro diagnostic kit including the same capable of amplifying signals while enhancing specific binding efficiency to a target substance by including nanoparticles.

### Background Art

An in vitro diagnostic device refers to a device including a reagent used as a medical device used for an in vitro diagnostic test, and since the test is performed in vitro, a physical burden is less than medical devices that are directly inserted into the human body.

Among in vitro diagnostic technologies, an immunoassay method is a technique for diagnosing diseases by rapidly measuring a target substance in vitro using a substance derived from the human body such as blood, urine, and body fluids and plays an important role in clinical decision making and becomes a required element in the treatment of patients.

The most representative immunoassay method is an enzyme-linked immunosorbent assay (ELISA), which may perform the immunoassay economically and simply as a method in which the efficiency of immunoassay is certified to be basically used for diagnosis of human and animal diseases up to date.

Generally, the ELISA includes a process of detecting one analyte or target substance using an antibody labeled with an enzyme and a substrate to be colored through this enzyme reaction and has an advantage of diagnosing diseases through a simple process. However, there has been a problem that since secondary reaction with the antibody linked to the enzyme is required, it takes a time and there is a burden to a monetary aspect as antibody production costs used in the secondary reaction. Further, since some of the primary reaction and the secondary reaction may not proceed, there is a problem in terms of sensitivity of diagnosis.

A fluorescence-linked immunosorbent assay (FLISA) is developed to overcome the problem of the ELISA. The FLISA is a test method for measuring the fluorescent sensitivity according to a degree in which antigen-antibody reaction occurs by binding fluorescence to an antibody binding to the target substance and has been used for detecting a secondary antibody attached with a fluorescent material by chemical bonds. First, as a marker of the ELISA, an enzyme was used, but the fluorescent material has been usually used in the present due to the development of subsequent detection technology. Such a fluorescent material has been used with various fluorescent molecules including Alexa Fluor-based, xanthene-based, and cyanine-based fluorescent materials.

In the ELISA, a chemical bonding method using a reaction of an amino group and an aldehyde group, a method of physically adsorbing proteins, and the like have been mainly used. In the case of the chemical bonding method, the activity of the immobilized protein is reduced according to the elapsed time after immobilization and thus, it is difficult to secure high sensitivity. In the case of the physical adsorption method, since it is difficult to control a directivity of the protein to be immobilized and maintain the protein activity after immobilization, it is difficult to secure sensitivity and reproducibility, and thus, it is difficult to be applied to protein analysis products that require trace quantitative analysis. Therefore, products for analyzing a high concentration target that is relatively low in the effect of this problem were mainly developed.

Further, the protein immobilized on a solid substrate has a lot of limitations to repeatedly reproducibly read a change in trace protein because denaturation of the immobilized protein and nonspecific interactions occur over time.

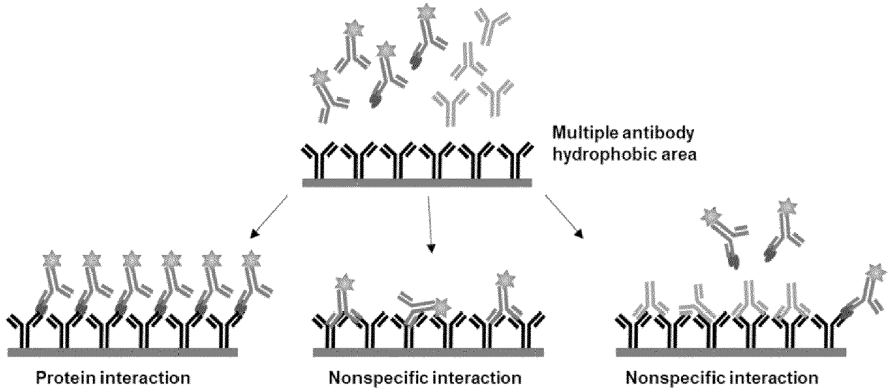

The ELISA and the FLISA are methods of directly immobilizing the proteins on the solid substrate, and have a problem that the denaturation of the immobilized proteins occurs over time and then nonspecific interactions between the proteins other than antigen-antibody reactions occur. In addition, during the measurement of trace protein (to 100 pg/ml), when the nonspecific sensitivity is included in the result, there is a case where a wrong result is shown at a high or low concentration of 10 to 1000 pg/ml or the like. For this reason, the assay methods based on the ELISA and the FLISA are suitable for analysis of a high concentration of protein which is not affected by the concentration due to nonspecificity, but in a very low concentration analysis, since there is a limitation that it is difficult to analyze actual sensitivity due to nonspecificity, it is difficult to secure the analysis technology with high reproducibility and repeatability.

The above-described technical configuration is the background art for helping in the understanding of the present invention, and does not mean a conventional technology widely known in the art to which the present invention pertains.

### Summary of the invention

An object of the present disclosure is to enhance binding specificity by removing a nonspecific reaction occurring by directly immobilizing proteins on a solid substrate by an enzyme-linked immunosorbent assay (ELISA) and a fluorescence-linked immunosorbent assay (FLISA) of the related arts.

Another object of the present disclosure is to provide an immunoconjugate with high sensitivity, accuracy, and reproducibility capable of detecting a trace target substance through signal amplification, a preparing method thereof, and an in vitro diagnostic reagent and an in vitro diagnostic kit including the same.

The objects to be solved by the present disclosure are not limited to the aforementioned object(s), and other object(s), which are not mentioned above, will be apparent to those skilled in the art from the following description.

An aspect of the present disclosure provides a highly sensitive immunoconjugate including a) a first nanoparticle immunoconjugate including a first nanoparticle-DNA conjugate and a first antibody-DNA conjugate, wherein a DNA fragment included in the first nanoparticle-DNA conjugate and a DNA fragment included in the first antibody-DNA conjugate are complementarily linked to each other; and b) a second nanoparticle immunoconjugate including a second nanoparticle-DNA conjugate and a second antibody-DNA conjugate, wherein a DNA fragment included in the second nanoparticle-DNA conjugate and a DNA fragment included in the second antibody-DNA conjugate are complementarily linked to each other.

The first antibody may specifically bind to a part of a target substance and the second antibody may specifically bind to the other part of the target substance.

The DNA fragment included in the first nanoparticle-DNA conjugate may have a complementary binding force with a DNA probe immobilized in a biochip.

The surface of each of the first nanoparticle and the second nanoparticle may include at least one functional group of an amine group (-NH₂) , a carboxyl group (-COOH) or an aldehyde group (-COH).

The first nanoparticle and the second nanoparticle may be latex beads.

The latex bead may have a diameter of 100 to 500 nm.

The second nanoparticle may contain a fluorescent material.

15,000 to 40,000 DNA fragments may bind to each of the first nanoparticle and the second nanoparticle.

Another aspect of the present disclosure provides a preparing method of a highly sensitive immunoconjugate including the steps of: (i) preparing a first nanoparticle-DNA conjugate and a second nanoparticle-DNA conjugate, (ii) preparing a first antibody-DNA conjugate and a second antibody-DNA conjugate; and (iii) preparing a first nanoparticle immunoconjugate and a second nanoparticle immunoconjugate in which a DNA fragment included in the first nanoparticle-DNA conjugate and a DNA fragment included in the first antibody-DNA conjugate are complementarily linked to each other and a DNA fragment included in the second nanoparticle-DNA conjugate and a DNA fragment included in the second antibody-DNA conjugate are complementarily linked to each other.

In the first nanoparticle-DNA conjugate and the second nanoparticle-DNA conjugate, an NH₂ group of a terminal of the DNA fragment may bind to a carboxyl group of the surface of each of the first nanoparticle and the second nanoparticle.

Yet another aspect of the present disclosure provides an in vitro diagnostic reagent including the highly sensitive immunoconjugate.

Still another aspect of the present disclosure provides an in vitro diagnostic kit including the highly sensitive immunoconjugate.

According to the present disclosure, there are effects of providing a highly sensitive immunoconjugate, a preparing method thereof, and an in vitro diagnostic reagent and an in vitro diagnostic kit including the same.

Further, according to the present disclosure, it is possible to remove nonspecific reactions occurring in the related arts and enhance the binding specificity by introducing DNA-DNA binding between a solid substrate and an immunoconjugate.

Further, according to the present disclosure, it is possible to detect a trace target substance at a pictogram level by binding multiple biomarkers of antibodies and genes to surfaces of nanoparticles or fluorescent nanoparticles to amplify detection signals and enhance the sensitivity, accuracy, and reproducibility.

### Brief description of the drawings

The above and other aspects, features and other advantages of the present invention will be more clearly understood from the following detailed description taken in conjunction with the accompanying drawings, in which:
FIG. 1 is a schematic diagram illustrating a highly sensitive immunoconjugate according to an embodiment of the present disclosure;
FIG. 2A is a schematic diagram illustrating a preparing method of a first antibody-DNA conjugate according to an embodiment of the present disclosure;
FIG. 2B is a schematic diagram illustrating a preparing method of a second antibody-DNA conjugate according to an embodiment of the present disclosure;
FIG. 3 is a schematic diagram illustrating a preparing method of an antibody-fluorescence immunoconjugate according to an embodiment of the present disclosure;
FIG. 4 is a schematic diagram illustrating an operational principle of an in vitro diagnostic kit including a highly sensitive immunoconjugate according to an embodiment of the present disclosure;
FIG. 5A is a schematic diagram illustrating a detection method using an enzyme-linked immunosorbent assay (ELISA) according to an embodiment of the present disclosure;
FIG. 5B illustrates a measurement concentration range of Troponin T of the ELISA according to an embodiment of the present disclosure;
FIG. 6A is a schematic diagram illustrating a fluorescence-linked immunosorbent assay (FLISA) using a primary immunoconjugate according to an embodiment of the present disclosure;
FIG. 6B illustrates a measurement concentration range of Troponin T of the FLISA using the primary immunoconjugate according to an embodiment of the present disclosure;
FIG. 7A is a schematic diagram illustrating a FLISA using a secondary (highly sensitive) immunoconjugate according to an embodiment of the present disclosure; and
FIG. 7B illustrates a measurement concentration range of Troponin T of the FLISA using the secondary (highly sensitive) immunoconjugate according to an embodiment of the present disclosure.

### Detailed description of the embodiments

Before describing the present disclosure in detail, terms or words used in this specification should not be construed as unconditionally limited to a conventional or dictionary meaning, and the inventors of the present disclosure can appropriately define and use the concept of various terms in order to describe their invention in the best method. Furthermore, it should be understood that these terms or words should be interpreted as meanings and concepts consistent with the technical idea of the present disclosure.

That is, the terms used in this specification are only used to describe a preferred embodiment of the present disclosure, and are not intended to specifically limit the contents of the present disclosure, and it should be noted that these terms are terms defined in consideration with various possibilities of the present disclosure.

In addition, in this specification, it should be understood that the singular expression may include a plural expression unless clearly indicated in another meaning in the context, and even if similarly expressed in the plural, the singular expression may include the meaning of the singular number.

Throughout this specification, when a component is described as "including" the other component, the component does not exclude any other component, but may further include any other component unless otherwise indicated in contrary.

Further, hereinafter, in the following description of the present disclosure, a detailed description of a configuration determined to unnecessarily obscure the subject matter of the present disclosure, for example, known technologies including the related arts may be omitted.

Hereinafter, the present disclosure will be described in more detail.

The present disclosure is to develop a method of binding antibody-DNA conjugates linked with antibody-DNA to a 9G DNA solid substrate immobilized with genes through DNA-DNA reaction, instead of a method of directly immobilizing antibody proteins on the solid substrate in order to solve a nonspecific problem occurring in low-concentration protein analysis in measurement methods based on ELISA and FLISA of the related arts.

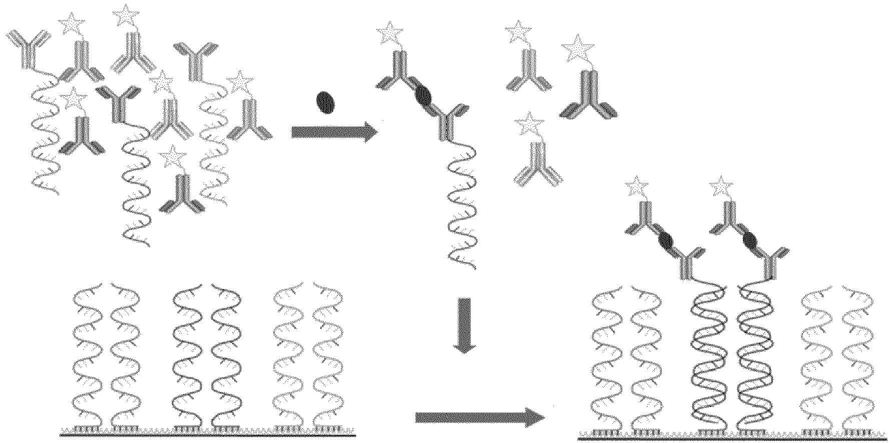

As such, since the 9G DNA substrate consisting of a DNA monomolecular layer has a hydrophilic surface, there is no nonspecific binding to other proteins, and since the antibody binding to the DNA of the solid substrate is also an antibody-DNA conjugate linked to the gene, there is no nonspecificity by the protein, so that low-concentration protein analysis is possible.

However, there is still a part to be solved in addition to the protein nonspecific problem to be established as an analysis system that accurately reproduces a very low concentration of biomarker material present in a sample.

In order to accurately detect the low concentration, a fluorescent marker used as an analysis marker needs to represent a stable and strong signal, but a single fluorescent molecule has limitations on a measurement method due to fluorescence signal intensity, fluorescent half-life, and the like, and technological development of new signal amplification capable of overcoming these disadvantages is required.

For signal-amplification technologies capable of increasing detection sensitivity by amplifying the analysis signal, the present inventors prepared an immunoconjugate including nanoparticles capable of very accurately measuring and analyzing traces of proteins and an in vitro diagnostic reagent and an in vitro diagnostic kit including the same.

Meanwhile, fluorescent nanoparticles have sizes of 1 micrometer or less unlike general phosphors having sizes of dozens of micrometers and exhibit different properties from single phosphors due to various effects occurring in a nano scale due to an increase in surface area/volume ratio according to the size. The fluorescent nanoparticles have features such as excellent thermal and chemical stability, excellent photostability, and high fluorescent intensity, and the like as compared with existing other fluorescent materials.

Under this underground, the present inventors prepared a highly sensitive immunoconjugate and an in vitro diagnostic reagent and an in vitro diagnostic kit including the same by binding biomarkers such as antibodies, genes, and the like with fluorescent nanoparticles by using fluorescent nanoparticles capable of compensating for the disadvantages of single fluorescent molecules as a medium, in order to develop a method capable of effectively detecting a target substance even if a smaller amount of sample is used by amplifying a fluorescent signal of a fluorescent immunodiagnostic kit.

### Highly sensitive immunoconjugate and preparing method thereof

A highly sensitive immunoconjugate of the present disclosure includes a) a first nanoparticle immunoconjugate including a first nanoparticle-DNA conjugate and a first antibody-DNA conjugate, wherein DNAs of the first nanoparticle-DNA conjugate and the first antibody-DNA conjugate are complementarily linked to each other; and b) a second nanoparticle immunoconjugate including a second nanoparticle-DNA conjugate and a second antibody-DNA conjugate, wherein DNAs of the second nanoparticle-DNA conjugate and the second antibody-DNA conjugate are complementarily linked to each other.

A preparing method of the highly sensitive immunoconjugate of the present disclosure includes the steps of (i) preparing a first nanoparticle-DNA conjugate and a second nanoparticle-DNA conjugate, (ii) preparing a first antibody-DNA conjugate and a second antibody-DNA conjugate; and (iii) preparing a first nanoparticle immunoconjugate and a second nanoparticle immunoconjugate in which DNA fragments of the first nanoparticle-DNA conjugate and the first antibody-DNA conjugate are complementarily linked to each other and DNA fragments of the second nanoparticle-DNA conjugate and the second antibody-DNA conjugate are complementarily linked to each other.

The highly sensitive immunoconjugate of the present disclosure includes the first nanoparticle immunoconjugate and the second nanoparticle immunoconjugate.

The first nanoparticle immunoconjugate includes the first nanoparticle-DNA conjugate and the first antibody-DNA conjugate, wherein the DNA fragments of the first nanoparticle-DNA conjugate and the first antibody-DNA conjugate may be complementarily linked to each other.

The first antibody may specifically bind to a part of the target substance and the second antibody may specifically bind to the other part of the target substance.

### <First nanoparticle-DNA conjugate>

In the first nanoparticle-DNA conjugate, DNA fragments bind to a first nanoparticle.

The first nanoparticles are preferably latex beads or latex particles as microsperes. The latex beads may be formed from an amorphous polymer such as polystyrene as spherical particles with a colloidal size. Because of a method of arranging polystyrene chains to the beads, since the surface is very hydrophobic, the latex beads are an ideal material to adsorption of a material such as proteins.

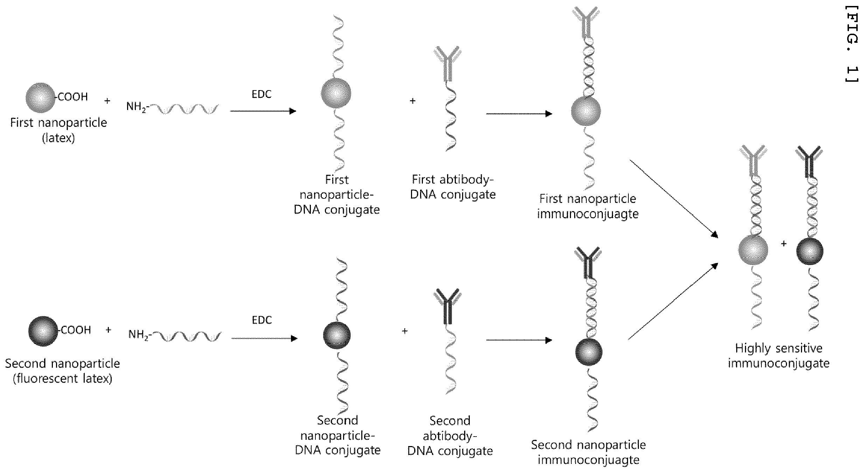

On the surface of the nanoparticle, a variety of surface modifications may be used to bind various molecules and proteins to the bead surface. The surface of the nanoparticle may include any one functional group of an amine group (-NH₂), a carboxyl group (-COOH) or an aldehyde group (-COH) . For example, the nanoparticle illustrated in FIG. 1 consists of a polystyrene microsphere as a carboxyl latex including a carboxyl group on a particle surface. In addition, an amine latex consists of a polystyrene microsphere and includes an amine group on the particle surface. Functional groups of the particle surface may be used for co-binding with components of genes, antigens, and antibodies. The size of the latex bead may be used with a diameter of 20 to 1000 nm, and preferably a diameter of 100 to 500 nm.

15,000 to 40,000 DNA fragments may bind to the nanoparticle.

Referring to FIG. 1, a NH₂-DNA fragment binds to a carboxyl group on the nanoparticle surface to form a nanoparticle-DNA conjugate. Accordingly, although not illustrated in FIG. 1, 15,000 or more of DNA fragments may be bound in the nanoparticle-DNA conjugate.

Accordingly, in the highly sensitive immunoconjugate according to the present disclosure, the detection sensitivity is increased by 100 times or more compared to the conventional ELISA, and the detection sensitivity is increased by 10 times or more compared to a single fluorescent immunoconjugate.

### <First antibody-DNA conjugate>

In the first antibody-DNA conjugate, DNA fragments bind to a first antibody.

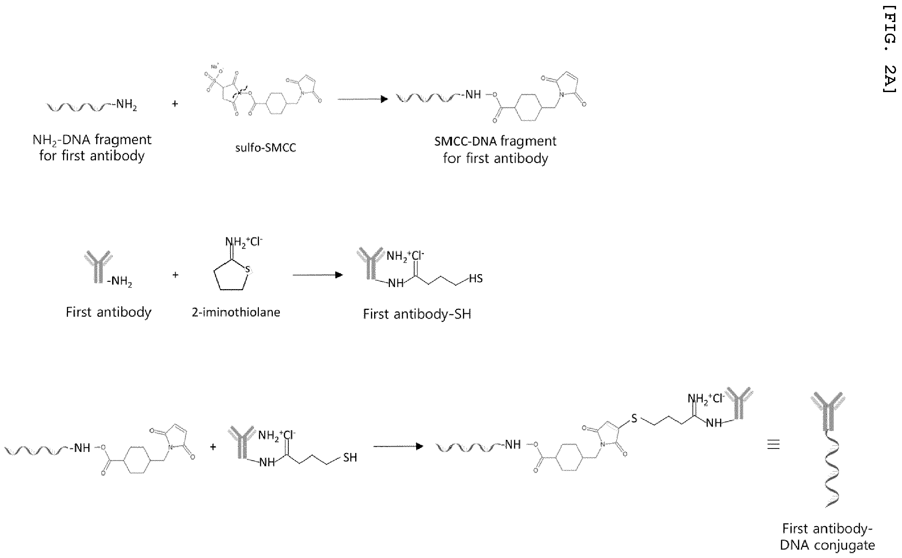

Referring to FIG. 2A, a first antibody-DNA conjugate is formed by binding a first antibody-SH to a SMCC-DNA fragment for the first antibody which is prepared by chemical bond of an NH₂-DNA fragment for the first antibody and sulfo-SMCC. The DNA fragment of the first SMCC-DNA fragment may be an oligonucleotide with a size of 30 to 40 mer.

The first antibody is an immunoglobulin which specifically binds to a target biomolecule for purification, detection, and measurement of a target substance.

The target substance includes all biomolecules such as a specific protein, an autoantibody, a viral phage, a nucleic acid molecule aptamer, hapten (DNP), and the like, and further, is not particularly limited to the detailed description.

### <First nanoparticle immunoconjugate>

Referring to FIG. 1, the first nanoparticle-DNA conjugate and the first antibody-DNA conjugate bind to each other to form the first nanoparticle immunoconjugate. The DNA fragments of the first nanoparticle-DNA conjugate and the first antibody-DNA conjugate include complementary sequences with each other to be hybridized and linked to each other. For example, the first NH₂-DNA fragment (the DNA fragment included in the first nanoparticle-DNA conjugate of FIG. 1) and the first SMCC-DNA fragment (the DNA fragment included in the first antibody-DNA conjugate of FIG. 1) of Table 2 have complementary base sequences with each other.

### <Second nanoparticle-DNA conjugate>

Referring to FIG. 1, the second nanoparticle-DNA conjugate may be prepared by the same preparing method as the first nanoparticle-DNA conjugate, except that the second nanoparticle is a fluorescent latex and except for the sequences of the DNA fragments.

The second nanoparticle is preferably a fluorescent latex containing a fluorescent material. The second nanoparticle may contain various fluorescent dyes therein. A fluorescence wavelength may use various fluorescences, such as (Ex/Em): Blue(365/415), Yellow-green(505/515), Nile red (535/575), Orange (540/560), Red-orange (565/580), Red(580/605), Crimson (625/645), Dark red (660/680), and Infrared (715/755). The material, the functional groups of the surface, the size, and the like of the second nanoparticle are duplicated with those described in the first nanoparticle, and will be omitted.

### <Second antibody-DNA conjugate>

In the second antibody-DNA conjugate, DNA fragments bind to a second antibody.

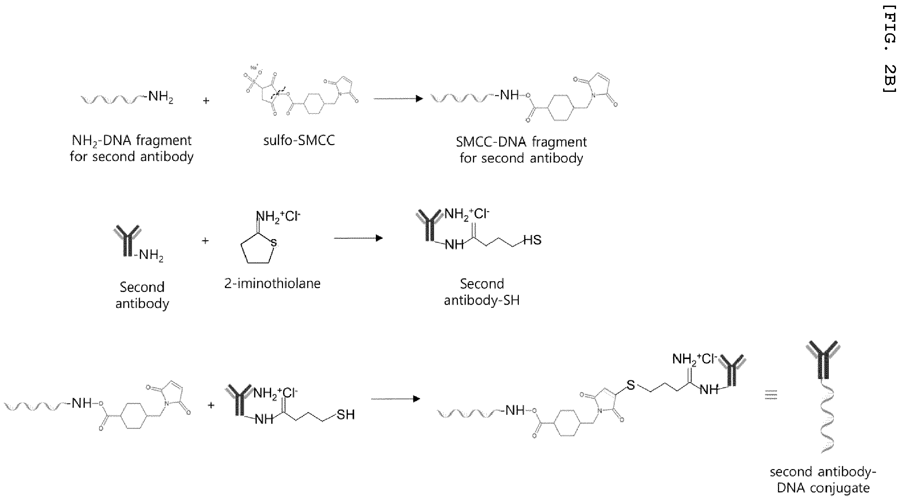

Referring to FIG. 2B, the preparing method of the second antibody-DNA conjugate may be the same as the preparing method of the first antibody-DNA conjugate, except for the sequences of the DNA fragments and the antibody.

The second antibody is immunoglobulin that specifically binds to a target biomolecule for purification, detection, and measurement of the target substance, and immunoglobulin that specifically binds to a different site from a site where the first antibody binds to a specific protein.

The target substance includes all biomolecules such as a specific protein, an autoantibody, a viral phage, a nucleic acid molecule aptamer, hapten (DNP), and the like, and further, is not particularly limited to the detailed description.

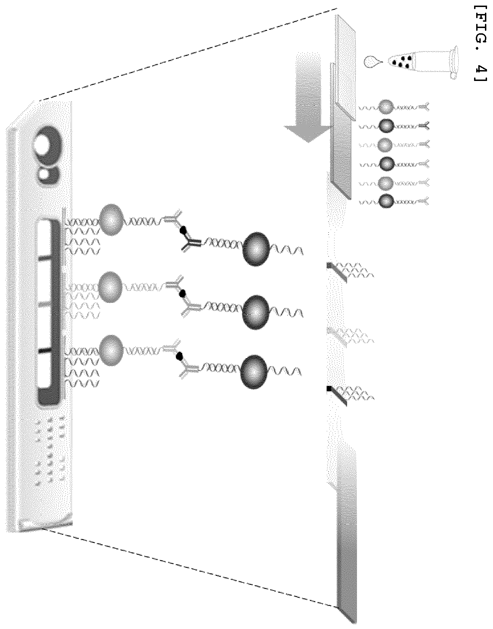

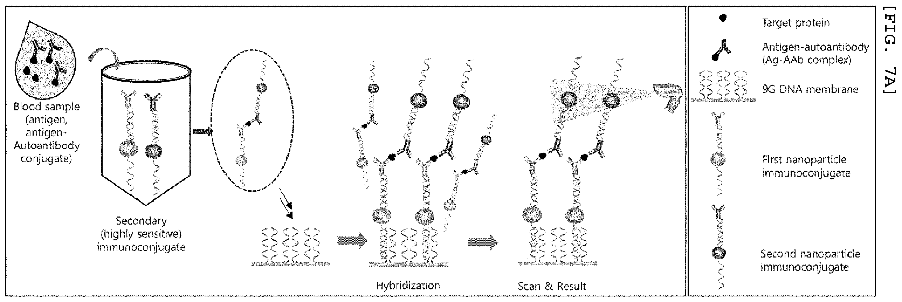

In the present disclosure, the first antibody specifically binds to a part of the target substance and the second antibody specifically binds to the other part of the target substance. Referring to FIG. 4 or 7A, the first antibody specifically binds to a part of one target substance and the second antibody binds to the other part of the same target substance. The target substance may be a target protein to be detected, but is not limited thereto.

### <Second nanoparticle immunoconjugate>

Referring to FIG. 1, the second antibody-DNA conjugate (prepared in FIG. 2) complementarily binds to the second nanoparticle-DNA conjugate in which a second NH₂-DNA fragment binds to a carboxyl group of the surface of the second nanoparticle to form the second nanoparticle immunoconjugate.

The DNA fragments of the second nanoparticle-DNA conjugate and the second antibody-DNA conjugate include complementary sequences with each other to be hybridized and linked to each other. For example, the second NH₂-DNA fragment (the DNA fragment included in the second nanoparticle-DNA conjugate of FIG. 1) and the second SMCC-DNA fragment (the DNA fragment included in the second antibody-DNA conjugate of FIG.

### 2) of Table 2 have complementary base sequences with each other. <Highly sensitive immunoconjugate>

The highly sensitive immunoconjugate of the present disclosure is prepared by combining the first nanoparticle immunoconjugate and the second nanoparticle immunoconjugate.

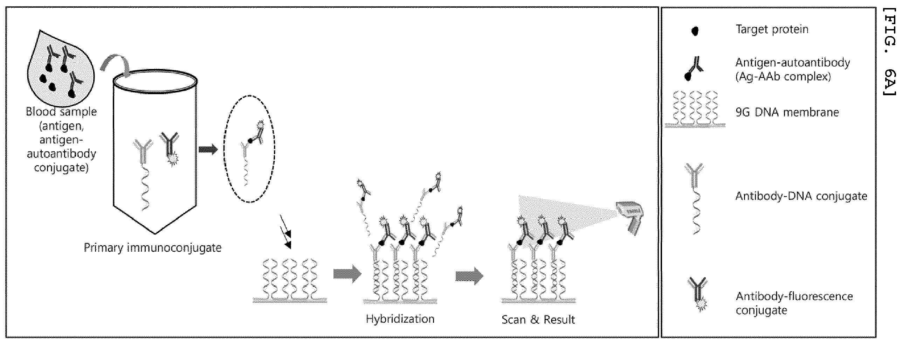

Referring to FIG. 7A, when the highly sensitive immunoconjugate of the present disclosure reacts with a specimen, a primary antibody and a secondary antibody bind to antigens in the specimen, and when this mixture flows on a biochip immobilized with a DNA probe, the DNA fragment of the nanoparticle binding to the first antibody complementarily binds to the DNA probe (DNA-DNA bond) and a signal is detected by a fluorescent nanoparticle linked to the second antibody. Since 15,000 to 40,000 DNA fragments may bind to the first nanoparticle and the second nanoparticle, the fluorescent signal is amplified to detect a target substance with high sensitivity. Referring to the following Examples, in the highly sensitive immunoconjugate according to the present disclosure, the detection sensitivity is increased by 100 times or more compared to the conventional ELISA, and the detection sensitivity is increased by 10 times or more compared to a single fluorescent immunoconjugate (see FIG. 6A).

### In vitro diagnostic reagent

The present disclosure provides an in vitro diagnostic reagent including the highly sensitive immunoconjugate.

The in vitro diagnostic reagent according to the present disclosure may further include a material which is commonly used in the art of the present disclosure. Particularly, the in vitro diagnostic reagent may further include a reaction buffer for an antigen-antibody reaction, a washing buffer used for washing after the reaction, and the like, but is not limited thereto.

### In vitro diagnostic kit

The present disclosure provides an in vitro diagnostic kit including the highly sensitive immunoconjugate.

The in vitro diagnostic kit according to the present disclosure may further include a device and a material which are commonly used in the art of the present disclosure. Particularly, the in vitro diagnostic kit may further include a biochip to which 9 guanines (9G) technique is applied, and a lateral flow using the fluorescence immunoassay is preferable, but is not limited thereto.

As an example of the in vitro diagnostic kit according to the present disclosure, referring to FIG. 4, the in vitro diagnostic kit includes a diagnostic strip including at least two or more test lines and a diagnostic kit body receiving the diagnostic strip. The diagnostic strip includes a sample pad into which a specimen including a target substance is injected, a first test line which is connected to the sample pad and immobilized with a first probe gene to which a first highly sensitive immunoconjugate specifically binds, and a second test line which is immobilized with a second probe gene to which a second highly sensitive immunoconjugate specifically binds. The second test line includes a glass fiber detection film fixed to a position spaced apart from the first test line.

Here, on the glass fiber detection film, the first test lien and the second test line are sequentially formed based on the sample pad side.

In addition, the diagnostic strip may further include an absorption pad which is attached to a support part while sequentially connected to the detection film and in which the remaining specimen passing through the detection film is absorbed.

In a diagnostic strip in a wide-range in vitro diagnostic kit applicable to various target substances, two or more test lines (DNA probes) specifically binding to the same target substance in the specimen are provided, so that quantification of the target substance in the specimen may be performed with high reliability even if the in vitro diagnosis is performed using a low concentration of specimen.

In the diagnostic strip in the in vitro diagnostic kit, two or more test lines specifically binding to the same target substance in the specimen are provided, so that a concentration range of the specimen capable of accurate quantification may be widely applied from a low concentration to a high concentration.

The vitro diagnostic kit according to the present disclosure may be widely applied to various diagnostics, such as pregnancy diagnosis, cardiovascular disease diagnosis, inflammation diagnosis, cancer diagnosis, and the like.

### Examples

Hereinafter, the present disclosure will be described in detail with reference to Examples for specific description. However, Examples according to the present disclosure may be modified in various forms, and it is not interpreted that the scope of the present disclosure is limited to the following Examples. Examples of the present disclosure will be provided for more completely explaining the present disclosure to those skilled in the art.

In the following Preparation Examples, Comparative Examples, Examples, and drawings, a 'primary immunoconjugate' means a combination of an antibody-DNA conjugate and an antibody-fluorescence conjugate and a 'secondary immunoconjugate' or 'highly sensitive immunoconjugate' means a combination of a first nanoparticle immunoconjugate and a second nanoparticle immunoconjugate.

### <Materials>

Materials used in immunoconjugates of the following Preparation Examples and Examples were shown in Table 1 and DNA sequences used in the immunoconjugates were shown in Table 2.

**[Table 1]**

| Item | Base sequence and feature |
|---|---|
| First nanoparticle (latex) | Microspere, latex bead or latex particle |
| Second nanoparticle (fluorescence) | Microspere, fluorescent bead or fluorescent particle |
| First antibody | Antibody binding to first part of specific protein as primary antibody |
| Second antibody | Antibody binding to second part of specific protein as primary antibody |

**[Table 2]**

| Item | Base sequence and feature | Size |
|---|---|---|
| NH₂-DNA fragment for first antibody | NH₂-TTTATATTTATACCTTGCGAGCGCGG | 26 |
| NH₂-DNA fragment for second antibody | NH₂-TTTATATTTTGGCCACACTGTCCATT | 26 |
| SMCC-DNA fragment for first antibody | SMCC-TTTATATTTCCGCGCTCGCAAGGTAT | 26 |
| SMCC-DNA fragment for second antibody | SMCC-TTTATATTTAATGGACAGTGTGGCCA | 26 |

### <Preparation Example 1> Antibody-DNA conjugate

FIGS. 2A and 2B illustrate schematic diagrams of preparing methods of a first antibody-DNA conjugate and a second antibody-DNA conjugate, and the preparing methods of the first antibody-DNA conjugate and the second antibody-DNA conjugate were the same as each other. Referring to FIGS. 2A and 2B, the antibody-DNA conjugate was prepared by preparing SMCC-DNA from NH₂-DNA (Step 1), introducing an -SH functional group to the antibody (Step 2), and forming the antibody-DNA conjugate by reaction of SMCC-DNA and antibody-SH (Step 3).

### <Preparation Example 1.1> Preparation of SMCC-DNA

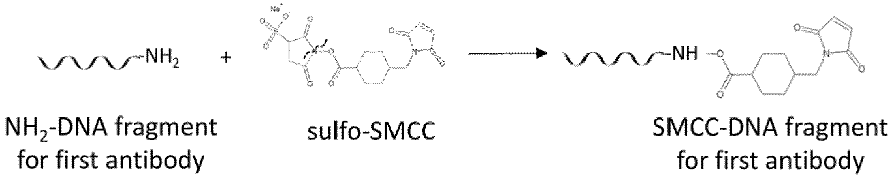

100 µl of NH₂-DNA (35 nmol) and 10 µl of sulfo-SMCC (10 mg/ml) were put in a 1.5 ml tube and reacted at room temperature for 1 hour. After the reaction, the remaining non-reacted sulfo-SMCC was removed through Sephadex LH20 column purification and only the SMCC-DNA was obtained to confirm the concentration.

### <Preparation Example 1.2> Preparation of antibody-SH

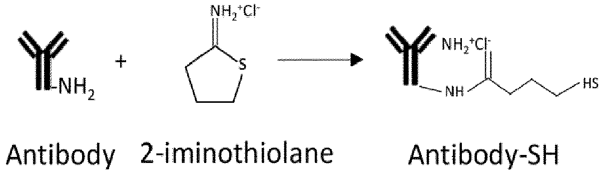

100 µl of an antibody (0.5 mg) and 1 mg of 2-iminothiolane powder were put in a tube and reacted at room temperature for 1 hour. After the reaction with the antibody-SH, the remaining iminothiolane was separated through a Sephadex LH20 column and the concentration of the antibody-SH obtained through the column was confirmed.

### <Preparation Example 1.3> Preparation of antibody-DNA conjugate

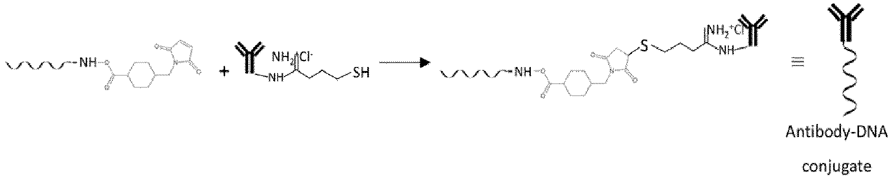

100 µl of 1 X PBS was put in a tube added with the SMCC-DNA fragment prepared in Preparation Example 1.1 and vortex-mixed, and then the antibody-SH prepared in Preparation Example 1.2 was added at a ratio of 1:1. After reaction at room temperature for 30 minutes, the mixture was purified through a Sephadex LH20 column to obtain an antibody-DNA conjugate.

### <Preparation Example 2> Nanoparticle immunoconjugate

FIG. 1 illustrates a schematic diagram of a preparing method of a nanoparticle immunoconjugate, and the preparing methods of the first nanoparticle immunoconjugate and the second nanoparticle immunoconjugate were the same as each other and there was a difference only in that the second nanoparticle was a fluorescent latex. Referring to FIG. 1, the nanoparticle immunoconjugate was prepared by preparing a nanoparticle-DNA conjugate (Step 1) and reacting the antibody-DNA conjugate prepared in Preparation Example 1 with the nanoparticle-DNA conjugate (Step 2).

### <Preparation Example 2.1> Preparation of first nanoparticle-DNA conjugate

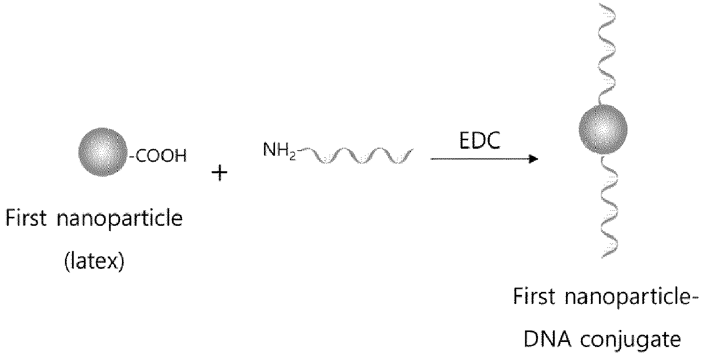

100 µl of a 2% nanoparticle and NH₂-DNA (20 nmole, 50 µl) were put in a tube and added with 200 µl of an MES buffer. 50 µl of EDC (200 mg/ml) was added and reacted at room temperature for 2 hours. After centrifugation at 13,000 rpm, a supernatant was removed to obtain a nanoparticle-DNA conjugate in a pellet form.

### <Preparation Example 2.2> Preparation of first nanoparticle immunoconjugate

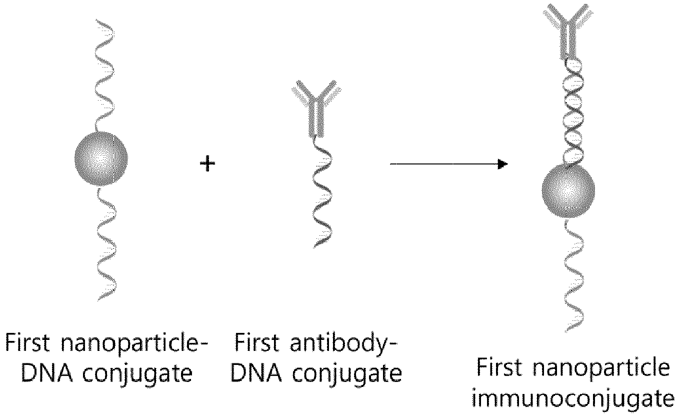

The first nanoparticle-DNA conjugate of Preparation Example 2.1 was dispersed in 1 ml of 1XPBS and vortexed, and then added with the first antibody-DNA conjugate (the DNA fragments of the first antibody-DNA conjugate and the first nanoparticle-DNA conjugate had the complementary sequences) prepared in Preparation Example 1 and reacted at room temperature for 30 minutes.

### <Preparation Example 2.3> Preparation of second nanoparticle immunoconjugate

Except for using a fluorescent latex as the second nanoparticle and the sequences of the DNA fragments and the antibody, Preparation Example 2.3 was performed in the same manner as Preparation Examples 2.1 and 2.2.

### <Example 1> Primary immunoconjugate

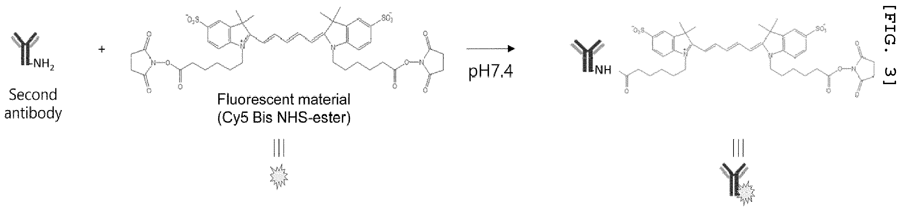

The antibody-DNA conjugate prepared in Preparation Example 1 and the antibody-fluorescence immunoconjugate prepared in FIG. 3 were mixed at 1:1 and used.

### <Example 2> Secondary (highly sensitive) immunoconjugate

The first nanoparticle immunoconjugate prepared in Preparation Example 2.2 and the second nanoparticle immunoconjugate prepared in Preparation Example 2.3 were mixed at 1:1 and used as a secondary (highly sensitive) immunoconjugate.

### <Comparative Example 1> Detection of troponin T by ELISA

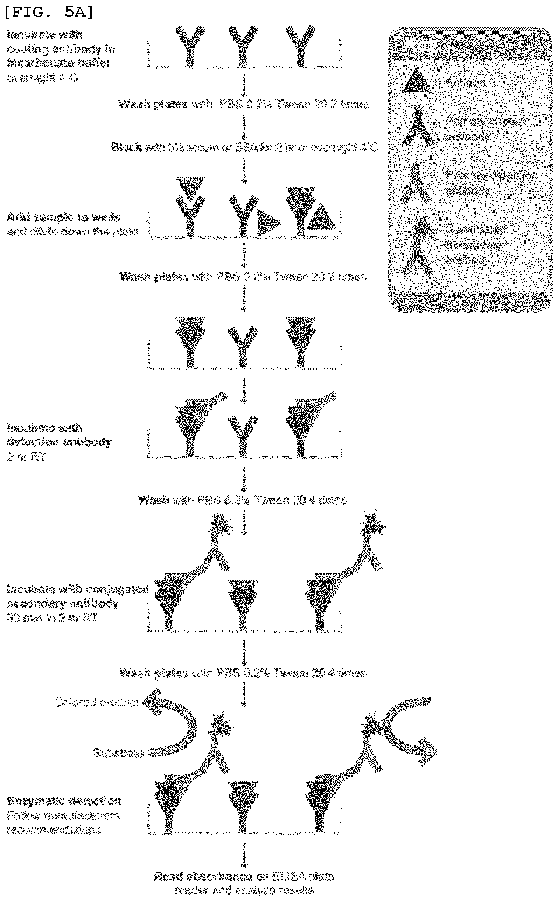

Human Cardiac Troponin T in the blood was detected by ELISA using an antibody-enzyme immunoconjugate. The ELISA was performed by the ELISA method illustrated in FIG. 5A.

### Coating of Troponin T capture antibody

1) A troponin T capture antibody was prepared at a concentration (1 to 10 µg/mL) using a carbonate/bicarbonate buffer (pH 9.6) and then dispensed in a PVC microtiter plate. 2) The plate cover was closed and the plate was incubated at 4°C for 12 hours. 3) The coating solution was removed and the plate was washed with 200 µL of PBS twice. 4) The plate was swept to remove the remaining washing solution. 5) The remaining solution was carefully removed with a paper towel.

### Blocking and sample injection

1) In order to block remaining protein-binding sites, 200 µL of a blocking buffer (5% non-fat dry milk/PBS) was added to each well of a coated well plate. 2) The well plate was incubated at room temperature for 2 hours. 3) Samples #1 to #7 were added in the well plate by 100 µL, respectively, and a sample for standards curve was added in the well plate by 100 µL. 4) The samples were incubated at 37°C for 90 min (an unknown sample was always compared with Standard Curve) . 5) The samples were removed and washed with 200 µL of PBS three times.

### Incubation for secondary antibody and detection

1) 100 µL of a diluted secondary antibody was added in each well. 2) It was confirmed whether the secondary antibody was linked to a different site from the capture antibody for a target protein. 3) The plate cover was closed and the well plate was incubated at room temperature for 2 hours. 4) The washing process was performed with PBS four times. 5) A detection antibody diluted with a blocking buffer was added in the well by 100 µL. 6) The plate cover was closed and the well plate was incubated at room temperature for 2 hours. 7) The washing process was performed with PBS four times.

### Detection

By using Horse Radish Peroxidase (HRP) as an enzyme for detection, the absorbance of a detection antibody-HRP immunoconjugate was measured. A TMB (3,3',5,5'-tetramethylbenzidine) solution was added to each well by 200 µl, incubated at room temperature for 30 minutes, and then added with 200 µl of a static solution (2 M H₂SO₄), and the absorbance of 450 nm was measured.

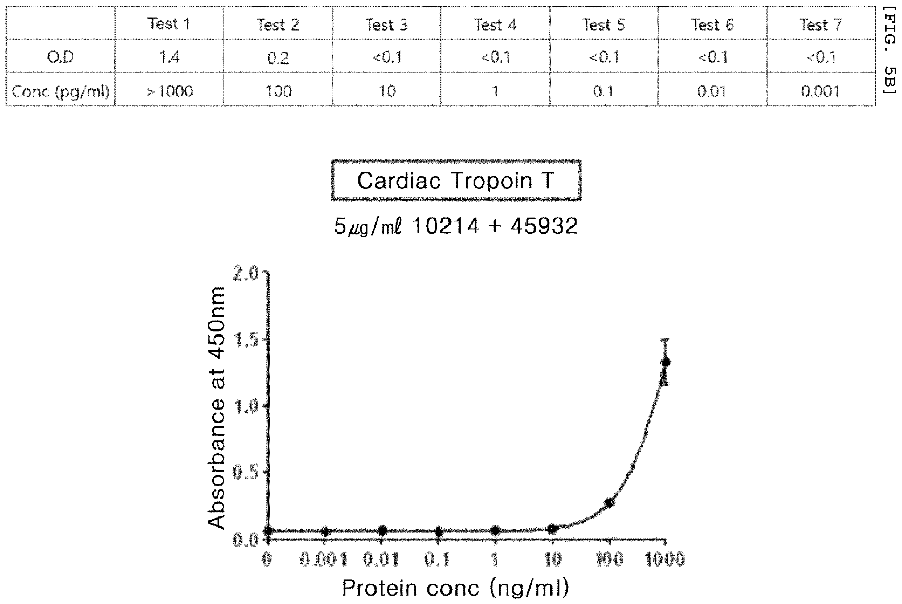

FIG. 5B illustrates a result of detecting cardiac Troponin T in a human blood sample by the ELISA method. Referring to FIG. 5B, it can seen that the measured cardiac troponin T concentration is shown in a detection range of 10 ng/ml (10,000 pg/ml) or more to about 1000 ng/ml in a high concentration area.

### <Experimental Example 1> Detection of troponin T using primary immunoconjugate

Troponin T was detected by a method of FIG. 6A using the primary immunoconjugate prepared in Example 1. 1) Blood plasma samples #1 to #7 were put in a microtube by 10 µl, respectively. 2) 100 µl of a primary immunoconjugate solution (mixture of primary immunoconjugate and antibody-fluorescence immunoconjugate) was incubated at room temperature for 10 minutes. 3) 60 µl of an R buffer was added and 170 µl of the mixture was deployed in a lateral flow membrane strip for 10 minutes. 4) 170 µl of a washing solution was added and the mixture was washed for 10 minutes. 5) The membrane strip was scanned by a membrane strip dedicated scanner (BMT 1D Scanner) and then the troponin T was detected.

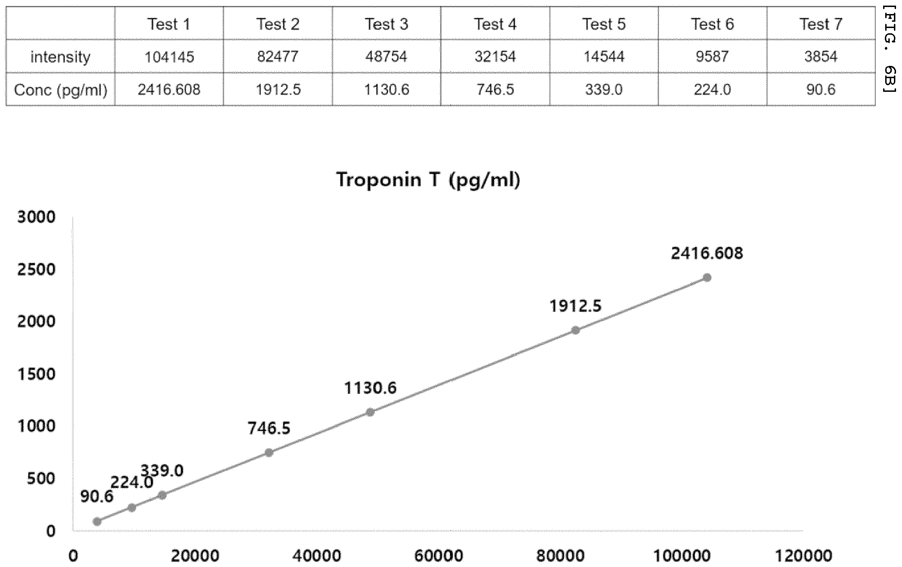

FIG. 6B is a result of detecting cardiac Troponin T in a human blood sample by lateral flow immunofluorescence assay using the primary immunoconjugate. Referring to FIG. 6B, it was confirmed that the measured cardiac troponin T concentration was shown in a detection range of 90 pg/ml to 2400 pg/ml in a high concentration area and the LOD was increased 10 times or more as compared with existing antibody-enzyme conjugate type of ELISA.

### <Experimental Example 2> Detection of troponin T using secondary (highly sensitive) immunoconjugate

Troponin T was detected by a method of FIG. 7A using the secondary (highly sensitive) immunoconjugate prepared in Example 2. 1) Blood plasma samples #1 to #7 were put in a microtube by 10 µl, respectively. 2) 100 µl of a secondary immunoconjugate solution was added and incubated at room temperature for 10 minutes. 3) 60 µl of an R buffer was added and 170 µl of the mixture was deployed in a lateral flow membrane strip for 10 minutes . 4) 170 □µl of a washing solution was added and the mixture was washed for 10 minutes. 5) The membrane strip was scanned by a membrane strip dedicated scanner (BMT 1D Scanner) and then the troponin T was detected.

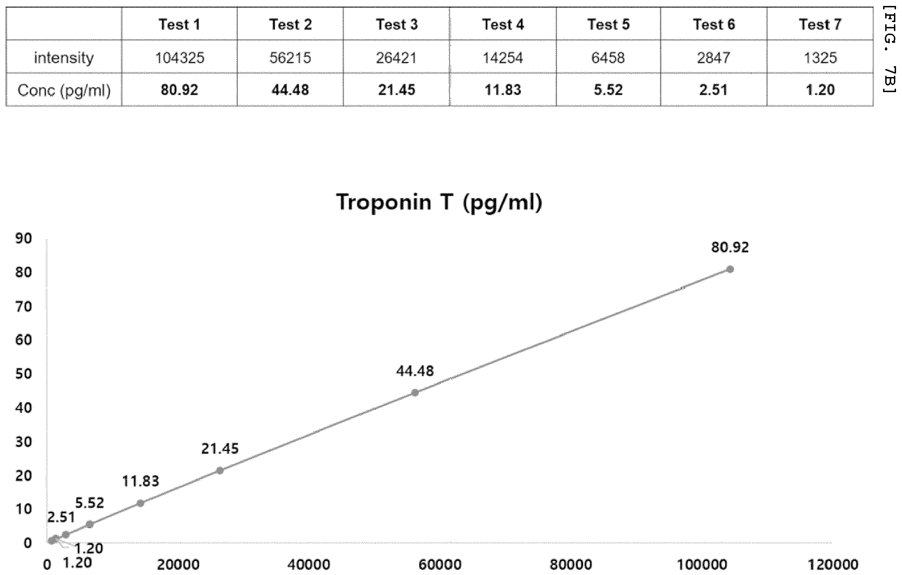

FIG. 7B is a result of detecting cardiac Troponin T in a human blood sample by lateral flow immunofluorescence assay using the secondary (highly sensitive) immunoconjugate. Referring to FIG. 7B, it was confirmed that the measured cardiac troponin T concentration was shown in a detection range of 1.2 pg/ml to 80 pg/ml in a low concentration area and the detection LOD was increased 100 times or more as compared with existing antibody-enzyme conjugate type of ELISA.

As described above, the detailed embodiments for the highly sensitive immunoconjugate according to the present disclosure, the preparing method thereof, and the in vitro diagnostic reagent and the in vitro diagnostic kit including the same had been described, but it will be apparent that various modifications can be made without departing from the scope of the present disclosure.

Therefore, the scope of the present disclosure should not be limited to the embodiments and should be defined by the appended claims and equivalents to the appended claims.

In other words, the embodiments described above are illustrative in all aspects and should be understood as not being restrictive, and the scope of the present disclosure is represented by appended claims to be described below rather than the detailed description, and it is to be interpreted that the meaning and scope of the appended claims and all changed or modified forms derived from the equivalents thereof are included within the scope of the present disclosure.

### <Explanation of sequence listing>

SEQ ID NO: 1 is a sequence of a DNA fragment included in a first nanoparticle-DNA conjugate according to an embodiment of the present disclosure.
SEQ ID NO: 2 is a sequence of a DNA fragment included in a second nanoparticle-DNA conjugate according to an embodiment of the present disclosure.
SEQ ID NO: 3 is a sequence of a DNA fragment included in a first antibody-DNA conjugate according to an embodiment of the present disclosure.
SEQ ID NO: 4 is a sequence of a DNA fragment included in

## Claims

1. A highly sensitive immunoconjugate comprising:
a) a first nanoparticle immunoconjugate including a first nanoparticle-DNA conjugate and a first antibody-DNA conjugate, wherein a DNA fragment included in the first nanoparticle-DNA conjugate and a DNA fragment included in the first antibody-DNA conjugate are complementarily linked to each other; and
b) a second nanoparticle immunoconjugate including a second nanoparticle-DNA conjugate and a second antibody-DNA conjugate, wherein a DNA fragment included in the second nanoparticle-DNA conjugate and a DNA fragment included in the second antibody-DNA conjugate are complementarily linked to each other.

2. The highly sensitive immunoconjugate of claim 1, wherein the first antibody specifically binds to a part of a target substance and the second antibody specifically binds to the other part of the target substance.

3. The highly sensitive immunoconjugate of claim 1, wherein the DNA fragment included in the first nanoparticle-DNA conjugate has a complementary binding force with a DNA probe immobilized in a biochip.

4. The highly sensitive immunoconjugate of claim 1, wherein the surface of each of the first nanoparticle and the second nanoparticle includes at least one functional group of an amine group (-NH₂), a carboxyl group (-COOH) or an aldehyde group (-COH).

5. The highly sensitive immunoconjugate of claim 1, wherein the first nanoparticle and the second nanoparticle are latex beads.

6. The highly sensitive immunoconjugate of claim 5, wherein the latex bead has a diameter of 100 to 500 nm.

7. The highly sensitive immunoconjugate of claim 1, wherein the second nanoparticle contains a fluorescent material.

8. The highly sensitive immunoconjugate of claim 1, wherein 15, 000 to 40, 000 DNA fragments bind to each of the first nanoparticle and the second nanoparticle.

9. A preparing method of a highly sensitive immunoconjugate comprising the steps of:
(i) preparing a first nanoparticle-DNA conjugate and a second nanoparticle-DNA conjugate;
(ii) preparing a first antibody-DNA conjugate and a second antibody-DNA conjugate; and
(iii) preparing a first nanoparticle immunoconjugate and a second nanoparticle immunoconjugate in which a DNA fragment included in the first nanoparticle-DNA conjugate and a DNA fragment included in the first antibody-DNA conjugate are complementarily linked to each other and a DNA fragment included in the second nanoparticle-DNA conjugate and a DNA fragment included in the second antibody-DNA conjugate are complementarily linked to each other.

10. The preparing method of the highly sensitive immunoconjugate of claim 9, wherein in the first nanoparticle-DNA conjugate and the second nanoparticle-DNA conjugate, an NH₂ group of a terminal of the DNA fragment binds to a carboxyl group of the surface of each of the first nanoparticle and the second nanoparticle.

11. An in vitro diagnostic reagent including the highly sensitive immunoconjugate of any one of claims 1 to 8.

12. An in vitro diagnostic kit including the highly sensitive immunoconjugate of any one of claims 1 to 8.

## Amended claims

### Amended claims in accordance with Rule 137(2) EPC.

1. A highly sensitive immunoconjugate comprising:
a) a first nanoparticle immunoconjugate including a first nanoparticle-DNA conjugate and a first antibody-DNA conjugate, wherein a DNA fragment included in the first nanoparticle-DNA conjugate and a DNA fragment included in the first antibody-DNA conjugate are complementarily linked to each other; and
b) a second nanoparticle immunoconjugate including a second nanoparticle-DNA conjugate and a second antibody-DNA conjugate, wherein a DNA fragment included in the second nanoparticle-DNA conjugate and a DNA fragment included in the second antibody-DNA conjugate are complementarily linked to each other,
wherein the DNA fragment included in the first nanoparticle-DNA conjugate has a complementary binding force with a DNA probe immobilized in a biochip,
wherein the second nanoparticle contains a fluorescent material,
wherein 15,000 to 40,000 DNA fragments bind to each of the first nanoparticle and the second nanoparticle.

2. The highly sensitive immunoconjugate of claim 1, wherein the first antibody specifically binds to a part of a target substance and the second antibody specifically binds to the other part of the target substance.

3. The highly sensitive immunoconjugate of claim 1, wherein the surface of each of the first nanoparticle and the second nanoparticle includes at least one functional group of an amine group (-NH₂), a carboxyl group (-COOH) or an aldehyde group (-COH).

4. The highly sensitive immunoconjugate of claim 1, wherein the first nanoparticle and the second nanoparticle are latex beads.

5. The highly sensitive immunoconjugate of claim 4, wherein the latex bead has a diameter of 100 to 500 nm.

6. A preparing method of a highly sensitive immunoconjugate comprising the steps of:
(i) preparing a first nanoparticle-DNA conjugate and a second nanoparticle-DNA conjugate;
(ii) preparing a first antibody-DNA conjugate and a second antibody-DNA conjugate; and
(iii) preparing a first nanoparticle immunoconjugate and a second nanoparticle immunoconjugate in which a DNA fragment included in the first nanoparticle-DNA conjugate and a DNA fragment included in the first antibody-DNA conjugate are complementarily linked to each other and a DNA fragment included in the second nanoparticle-DNA conjugate and a DNA fragment included in the second antibody-DNA conjugate are complementarily linked to each other,
wherein the DNA fragment included in the first nanoparticle-DNA conjugate has a complementary binding force with a DNA probe immobilized in a biochip,
wherein the second nanoparticle contains a fluorescent material,
wherein 15,000 to 40,000 DNA fragments bind to each of the first nanoparticle and the second nanoparticle.

7. The preparing method of the highly sensitive immunoconjugate of claim 6, wherein in the first nanoparticle-DNA conjugate and the second nanoparticle-DNA conjugate, an NH₂ group of a terminal of the DNA fragment binds to a carboxyl group of the surface of each of the first nanoparticle and the second nanoparticle.

8. An in vitro diagnostic reagent including the highly sensitive immunoconjugate of claim 1 to 5.

9. An in vitro diagnostic kit including the highly sensitive immunoconjugate of claim 1 to 5.
